# EUROPEAN PATENT SPECIFICATION

(11) **EP 3 597 789 B1**
(45) Date of publication and mention of the grant of the patent: **24.08.2022**
(21) Application number: 18767277.9
(22) Date of filing: 14.03.2018
(51) Int. Cl.: C23C 14/06, C23C 14/30, A61L 27/12, G02B 1/10

(54) **HYDROPHILIC VAPOR DEPOSITION FILM AND VAPOR DEPOSITION MATERIAL**
HYDROPHILER DAMPFABSCHEIDUNGSFILM UND DAMPFABSCHEIDUNGSMATERIAL
FILM HYDROPHILE DE DÉPÔT EN PHASE VAPEUR ET MATÉRIAU DE DÉPÔT EN PHASE VAPEUR

(30) Priority: 15.03.2017 JP 2017050448
(43) Date of publication of application: 22.01.2020
(73) Proprietor: Canon Optron Inc., Yuki-shi, Ibaraki 307-0015 (JP)
(72) Inventor: WATARAI, Takanori, Yuki-shi Ibaraki 307-0015 (JP); TAMEKUNI, Takahiro, Yuki-shi Ibaraki 307-0015 (JP); UESHIMA, Soichiro, Yuki-shi Ibaraki 307-0015 (JP); NUMAZAWA, Hiroto, Yuki-shi Ibaraki 307-0015 (JP); HORIE, Yukihiro, Yuki-shi Ibaraki 307-0015 (JP)
(74) Representative: TBK
(86) International application number: PCT/JP2018/010070
(87) International publication number: WO 2018/168963

(56) References cited:
- JP-A- S55 154 351
- JP-A- 2001 226 633
- JP-A- 2003 231 827
- US-A1- 2009 304 761
- US-B1- 6 344 276
- JUNGJIN PARK ET AL: "Methanol oxidation in nanostructured platinum/cerium-phosphate thin films", CURRENT APPLIED PHYSICS, ELSEVIER, AMSTERDAM, NL, vol. 11, no. 4, 8 April 2011 (2011-04-08), pages S2-S5, XP028334394, ISSN: 1567-1739, DOI: 10.1016/J.CAP.2011.07.005 [retrieved on 2011-07-12]
- DONGGI AHN ET AL: "Electrochemical stability in cerium-phosphate-coated LiCoO 2 thin films", JOURNAL OF MATERIALS RESEARCH, vol. 22, no. 3, 1 March 2007 (2007-03-01), pages 688-694, XP055756875, US ISSN: 0884-2914, DOI: 10.1557/jmr.2007.0092
- GABRIELA GRAZIANI ET AL: "A Review on Ionic Substitutions in Hydroxyapatite Thin Films: Towards Complete Biomimetism", COATINGS, vol. 8, no. 8, 1 August 2018 (2018-08-01), page 269, XP055756259, CH ISSN: 2079-6412, DOI: 10.3390/coatings8080269
- Adzila Sharifah ET AL: "Doping Metal into Calcium Phosphate Phase for Better Performance of Bone Implant Materials", Recent Patents on Materials Science, 4 January 2012 (2012-01-04), pages 18-47, XP055892515, DOI: 10.2174/1874465611205010018 Retrieved from the Internet: URL:http://www.eurekaselect.com/article/39 871 [retrieved on 2022-02-16]

## Description

### [Technical Field]

The present invention relates to a hydrophilic vapor deposition film and a vapor deposition material for producing the hydrophilic vapor deposition film.

### [Background Art]

A deposition method is a method of producing a thin film on the surface of a substrate, and a film can be formed on a substrate with any shape such as a lens, a lens cover, an automobile component, and a film. Since the film thickness can be precisely controlled on a nano order, it is easy to produce an optical multilayer film such as an antireflective film and a mirror. In addition, since a strong film can be formed without heating a substrate and the like, a film formation method is used in many industrial fields.

Phosphate compounds are widely used as biomaterials such as artificial bones and food additives because they are not harmful to the human body and have high biocompatibility, and they can be used for hydrophilic films by utilizing the hydrophilic performance that the materials have. Although an example in which a photocatalytic function of titanium oxide is applied for a hydrophilic film is known, since ultraviolet light is required for a photocatalyst, the function becomes weak indoors or at night. Therefore, it is thought that a hydrophilic film made of a phosphate compound which does not have such a restriction would be able to be widely used. In addition, phosphate compounds can also be used as glass materials because they melt at a low temperature and have low viscosity.

PTL 1 describes a film formation method of a hydrophilic film in which a surface treatment agent containing phosphoric acid or derivatives thereof, boric acid or derivatives thereof, and a solvent are applied and then heated.

US 2009/304761 A1 relates to a biocompatible coating comprising calcium phosphate that is functionally graded across the thickness of the coating.

US 6 344 276 B1 relates to a medical implant coated with an amorphous hydroxyapatite/titanium surface coating layer and the a surface coating is deposited onto a substrate from a composite target comprising 10-75% by volume of titanium and 90-25% by volume of hydroxyapatite.

Jungjin Park et al. (Methanol oxidation in nanostructured platinum/cerium-phosphate thin films, Current Applied Physics, Volume 11, Issue 4, Supplement, 2011, Pages S2-S5) relates to nanostructured Pt/CePO₄ thin-film electrodes consisting of the Pt nanocrystallites and amorphous CePO₄, which were synthesized by sputtering, and the enhanced electro-oxidation properties of methanol were investigated.

Ahn, D., Kim, C., Lee, JG. et al. (Electrochemical stability in cerium-phosphate-coated LiCoO2 thin films, Journal of Materials Research 22, 688-694 2007) relates to LiCoO₂ thin films having deposited a cerium-phosphate coating in order to improve the electrochemical stability of LiCoO₂ thin films.

### [Citation List]

### [Patent Literature]

[PTL 1]
Japanese Patent Application Publication No. H08-283042

### [Summary of Invention]

### [Technical Problem]

Although phosphate compounds have an advantage of high hydrophilic performance, they also have a disadvantage of low water resistance at the same time. Therefore, it is desirable to improve durability in order to expand applications such as outdoor use in consideration of practicality.

PTL 1 describes a film formation method of a hydrophilic film in which, in order to improve durability of a phosphoric acid film, a surface treatment agent containing phosphoric acid or derivatives thereof, boric acid or derivatives thereof, and a solvent is applied and then heated. However, it is difficult to apply a surface treatment agent to a substrate having a shape with a large curvature such as a plastic cover used for a network camera with a uniform film thickness, and the film cannot be used for an optical thin film for which the film thickness needs to be controlled. In addition, since a heat treatment is necessary after application, it is not possible to perform formation of a film on a substrate vulnerable to heat such as a plastic.

The present invention provides a hydrophilic vapor deposition film that has high water resistance and that can be formed on a substrate having a large curvature, a substrate having a complicated shape, or a substrate of which heating to a high temperature is difficult. In addition, the present invention provides a hydrophilic vapor deposition film of which the film thickness can be controlled precisely and the refractive index is stable.

### [Solution to Problem]

The present invention relates to a hydrophilic vapor deposition film as defined in claim 1.

In addition, the present invention relates to a vapor deposition material as defined in claim 2.

### [Advantageous Effects of Invention]

According to the present invention, it is possible to provide a hydrophilic vapor deposition film that has high water resistance and that can be formed on a substrate having a large curvature, a substrate having a complicated shape, or a substrate of which heating to a high temperature is difficult. In addition, it is possible to provide a hydrophilic vapor deposition film of which the film thickness can be controlled precisely and the refractive index is stable.

### [Brief Description of Drawings]

[Fig. 1]

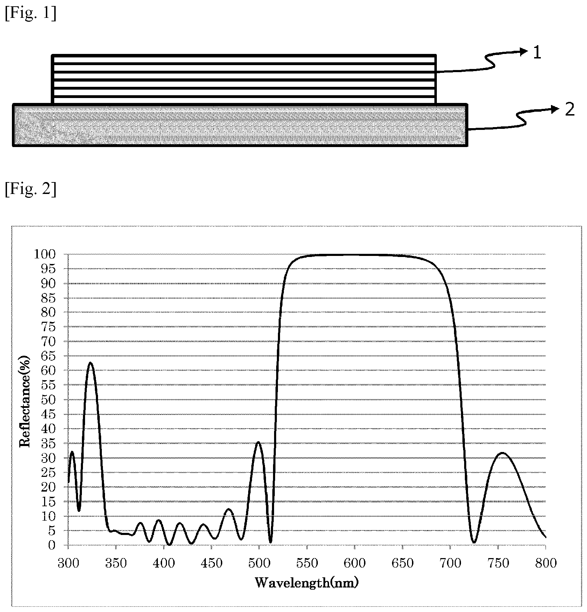

   Fig. 1 is a conceptual diagram of an optical mirror having a hydrophilic vapor deposition film on the outermost surface.
[Fig. 2]
   Fig. 2 is a diagram showing optical characteristics of a 17-layer optical mirror using a hydrophilic vapor deposition film on the outermost surface.

### [Description of Embodiments]

In the present invention, the descriptions "at least XX and not more than YY" and "XX to YY" representing numerical ranges indicate numerical ranges including the lower limit and the upper limit serving as end points unless otherwise specified.

Effects such as hydrophilic performance and water resistance of a hydrophilic vapor deposition film of the present invention and a vapor deposition material for forming the film will be described below in detail.

Since phosphoric acid is a water-soluble substance, it is a substance having high affinity for water and has hydrophilicity. When a thin film is produced using phosphoric acid, phosphoric acid alone has high solubility in water. Therefore, a thin film obtained from phosphoric acid maintains its hydrophilic performance immediately after film formation, but when it is exposed to rain outdoors, phosphoric acid may gradually dissolve in water that is adhered to the surface of the hydrophilic film, and it may not be possible to maintain the hydrophilic performance.

Thus, the inventors thought that, since phosphoric acid remains as trivalent ions PO₄³⁻ in water, when phosphoric acid and a metal capable of becoming trivalent that has a strong bonding strength with respect to phosphoric acid are caused to be present in a hydrophilic film obtained in the deposition method, phosphoric acid in the film will bind to a metal, dissolution of phosphoric acid in water will be limited, and the water resistance of the hydrophilic vapor deposition film will be improved.

In addition, it is found that, when a method in which a material mixture containing phosphoric acid and a metal capable of becoming trivalent is used for deposition and a thin film is formed is used, film formation on a substrate with any shape and material is possible. In addition, it is found that, since it is not necessary to heat a substrate to a high temperature, it is possible to extend the range of target substrates for which a hydrophilic vapor deposition film can be used.

For film formation according to a deposition method, known procedures can be used. For example, a substrate on which a film is formed and a vapor deposition material for forming a film may be put into a vacuum device, the vapor deposition material heated after evacuation, the vapor deposition material vaporized, and this vapor deposited on the substrate, thereby a film being obtained.

The method of producing a hydrophilic vapor deposition film of the present invention includes a process of vaporizing a vapor deposition material and depositing it on a substrate.

Regarding the substrate on which a film can be formed in the present invention, an object on which another thin film is filmed can also be used as a substrate regardless of its material or shape such as a glass, a plastic molded article, and a film.

In addition, a preferable aspect of the present invention is a multilayer film including a hydrophilic vapor deposition film on a substrate and in which the hydrophilic vapor deposition film is used as a multilayer film formed on the outermost surface.

It is possible to control the film thickness of a thin film to be formed on a nano order, and it is possible to produce a multilayer film such as a mirror as an optical thin film. For example, the film thickness of the hydrophilic vapor deposition film is preferably 5 to 200 nm, and more preferably 25 to 100 nm.

Even if the material is a mixture, film formation according to a deposition method is possible, but not all substances are evaporated at the same time, and there are substances that immediately evaporate after a material is heated and substances that do not evaporate easily unless heating is performed to a high temperature, depending on the melting point and evaporation temperature specific to a constituent substance.

The vapor deposition material that is used in the present invention includes at least phosphorus, oxygen, and two or more metal elements as constituent elements as constituent elements, wherein at least one of the metal elements is a metal element capable of becoming trivalent.

Regarding a raw material for the vapor deposition material, a phosphate compound is used. Phosphoric acid, which is a component that exhibits hydrophilicity, is in a liquid state at normal temperature, and cannot be directly used as a vapor deposition material. In addition, when phosphoric acid and other materials are mixed, the mixture becomes a paste form, and it has disadvantages of difficulty in removal from a mixing container, and deterioration of formability. In addition, since a large amount of water vapor is generated according to condensation of phosphoric acid during baking, there is concern of a baking device wearing. Therefore, regarding a phosphate material used in the present invention, a phosphate compound that is in a solid state at normal temperature such as calcium phosphate and aluminum phosphate is preferable.

The phosphate compound is not particularly limited, and those in various forms can also be used. Examples of phosphate compounds include aluminum phosphate, calcium phosphate represented by hydroxyapatite, cerium phosphate, magnesium phosphate, sodium phosphate, potassium phosphate, ammonium phosphate, barium phosphate, cobalt phosphate, lithium phosphate, boron phosphate, yttrium phosphate, and iron phosphate.

It is thought that, when a vapor deposition material is produced using aluminum phosphate and is used to form a film in a vacuum, since aluminum is a trivalent metal, it has strong bonding strength with respect to phosphoric acid, and the water resistance can be improved. However, when aluminum phosphate is heated, it decomposes into phosphorus pentoxide and aluminum oxide near 1,450°C. Since phosphorus pentoxide has a property of sublimating near 360°C in an atmospheric pressure atmosphere, phosphorus pentoxide generated by heating in a vacuum device quickly volatilizes, and is deposited on the substrate.

On the other hand, aluminum oxide formed at the same time has an evaporation temperature of about 2,000°C to 2,200°C in a vacuum atmosphere (1.0×10⁻³ Pa), and does not volatilize and continues to remain in the material. Therefore, the hydrophilic vapor deposition film formed does not contain aluminum, and becomes a hydrophilic vapor deposition film containing phosphorus and oxygen. As described above, since a hydrophilic vapor deposition film containing only phosphoric acid cannot maintain the hydrophilic performance outdoors, it is not possible to obtain a hydrophilic vapor deposition film having excellent durability by simply using aluminum phosphate.

In addition, when a vapor deposition material is produced using hydroxyapatite as a raw material and is used to form a film in a vacuum, it is possible to obtain a hydrophilic deposition film containing phosphorus, oxygen, and calcium. However, this hydrophilic vapor deposition film has high solubility in water like a film containing phosphoric acid, and has a hydrophilic performance immediately after film formation, but when it is exposed to rain outdoors, both phosphoric acid and calcium oxide gradually dissolve in water. As a result, the hydrophilic film gradually disappears and it is not possible to maintain the hydrophilic performance.

Therefore, in consideration of fixation of phosphoric acid described above, the inventors produced a vapor deposition material in which a metal compound such as an oxide and a fluoride containing a metal which is capable of becoming trivalent and has a strong bonding strength with respect to phosphoric acid is added to phosphate compounds including aluminum phosphate and hydroxyapatite as a raw material for a vapor deposition material. In addition, it is found that a hydrophilic vapor deposition film containing phosphoric acid and a trivalent metal obtained by depositing the material has improved water resistance and can maintain a hydrophilic performance for a long time.

The compound containing a metal capable of becoming trivalent mixed with phosphate is at least one selected from the group consisting of cerium compounds, iron compounds, gallium compounds, and bismuth compounds. Since all the metals in the compounds listed here are likely to become trivalent ions and have an ionic radius and a covalent bond radius similar to those of aluminum, they are thought to strongly bond to phosphate ions, and improvement in water resistance of a hydrophilic film can be expected therewith.

However, all compounds containing a metal capable of becoming trivalent do not contribute to improving water resistance of the hydrophilic vapor deposition film composed of a phosphate compound. Among the compounds, there are substances having a high evaporation temperature and substances having a low evaporation temperature. When a mixture with a phosphate compound is evaporated, it is preferable that an evaporation temperature of the compound be close to a thermal decomposition temperature of a phosphate compound. In the case of compounds having a significantly higher evaporation temperature than a decomposition temperature of a phosphate compound such as yttrium oxide and scandium oxide, phosphoric acid evaporates earlier and it is difficult to incorporate a metal capable of becoming trivalent or hexavalent into a phosphoric acid deposition film as in the description for aluminum phosphate.

On the other hand, in the case of a compound having a significantly lower evaporation temperature than a decomposition temperature of a phosphate compound such as molybdenum oxide, the compound evaporates before phosphate compound decomposes, and it is difficult to successfully incorporate a metal capable of becoming trivalent or hexavalent into a phosphoric acid deposition film, or it evaporates preferentially to a phosphate compound, a metal element concentration in the film becomes too high, and thus a problem of hydrophilic performance being degraded is caused.

Therefore, the evaporation temperature of a compound containing a metal capable of becoming trivalent or hexavalent in a vacuum state (1.0×10⁻³ Pa) is preferably ±500°C and more preferably ±350°C with respect to the thermal decomposition temperature of the phosphate compound.

Regarding examples of metal compounds such as oxides and fluorides containing a metal capable of becoming trivalent with the above preferable conditions, preferable examples of compounds containing a metal capable of becoming trivalent include cerium oxide and cerium fluoride, iron compounds such as iron oxide, gallium compounds such as gallium oxide, and bismuth compounds such as bismuth oxide. These are preferable because they evaporate at the same time as phosphoric acid, and are easily incorporated into a hydrophilic vapor deposition film.

That is, a compound containing a metal capable of becoming trivalent contained in a vapor deposition material is at least one selected from the group consisting of cerium compounds such as cerium oxide and cerium fluoride, iron compounds, gallium compounds, and bismuth compounds. At least one selected from the group consisting of cerium oxide and cerium fluoride is preferable.

In addition, a metal element capable of becoming trivalent contained in a hydrophilic vapor deposition film is at least one selected from the group consisting of cerium, iron, gallium, and bismuth. Cerium is particularly preferable.

Regarding a method of producing a vapor deposition material, for example, the following method can be used. A powder of a phosphate compound such as calcium phosphate and a powder of a compound containing a metal capable of becoming trivalent are mixed to obtain a mixture. Next, the mixture is granulated, molded, and sintered to obtain a vapor deposition material. When a phosphate of a metal capable of becoming trivalent such as cerium phosphate is used as a raw material, it may be granulated and molded alone.

For mixing and granulation of raw materials, a known mixer such as a ball mill can be used. During granulation, a known binder such as an acrylic binder may be used. An amount of the binder added is preferably about 5 to 20 parts by mass with respect to a total of 100 parts by mass of the phosphate compound and the compound containing a metal capable of becoming trivalent.

For molding, a known device such as a uniaxial pressure molding machine can be used. A pressure applied during molding is preferably in a range of 300 to 400 kgf/cm. For sintering, preferably, baking is performed, for example, under conditions of 400 to 1,700°C for about 1 to 4 hours. More preferably, baking is performed under conditions of 800°C to 1,400°C for 2 to 3 hours.

A thin film obtained using the vapor deposition material contains phosphorus, oxygen, and a metal capable of becoming trivalent. The thin film can be used as a hydrophilic film for a long time because it exhibits a hydrophilic performance and also has water resistance performance.

In order to improve water resistance of the hydrophilic vapor deposition film, the content of the metal element capable of becoming trivalent in the hydrophilic vapor deposition film is 0.7 to 80.0 mass% based on a total amount of phosphorus and metal elements contained in the hydrophilic vapor deposition film excluding oxygen. The content is preferably about 1.0 to 55.0 mass%, further preferably 3.0 to 30.0 mass%, and particularly preferably 4.0 to 30.0 mass%. When the content of the trivalent metal element is 0.7 mass% or more, since there is a sufficient amount of metal that bonds with phosphoric acid, phosphoric acid is unlikely to elute from the hydrophilic vapor deposition film and the hydrophilic film has favorable water resistance.

In addition, when the content is 80.0 mass% or less, since there is a sufficient amount of phosphoric acid groups that exhibit hydrophilicity in the film, the contact angle is reduced and a suitable hydrophilic performance is obtained.

On the other hand, the content of phosphorus in the hydrophilic vapor deposition film is 19.0 to 99.0 mass%.

The content of the metal element capable of becoming trivalent in the hydrophilic vapor deposition film can be measured by the following method.

### <Method of analyzing components in deposition film>

An example of analyzing components in a hydrophilic vapor deposition film containing, for example, P, Ca, and Ce, is shown below.

X-ray fluorescence intensities of P-Kα rays, Ca-Kα rays, and Ce-Lα rays excited from the surface of a polycarbonate substrate on which a hydrophilic vapor deposition film is formed are measured. A wavelength dispersion type ZSX Primus II (commercially available from Rigaku Corporation) is used as a device, and a side window type Rh anticathode X-ray tube is used as an X-ray tube. Other measurement conditions are summarized in Table 1. Regarding a quantitative method, a fundamental parameter (FP) method is used.

**[Table 1]**

| Element | Rh tube | | Crystal | Detector | 2 *θ* angle [° ] |
|---|---|---|---|---|---|
| | kV | mA | | | |
| P | 30 | 100 | G e | P C | 141.190 |
| C a | 40 | 75 | L i F 1 | PC | 113.120 |
| C e | 50 | 60 | L i F 1 | S C | 78.980 |

In order to set the content of the metal capable of becoming trivalent in the hydrophilic vapor deposition film to be within the above range, the content of the metal element capable of becoming trivalent in the vapor deposition material is preferably 1.5 to 42.0 mass% based on a total amount of phosphoric acid and metal elements contained in the vapor deposition material. The content is more preferably 2.0 to 35.0 mass%.

When the content is 1.5 mass% or more, a sufficient amount of metal can be incorporated into the deposition film. On the other hand, when the content is 42.0 mass% or less, an amount of phosphoric acid in the deposition film is sufficient, and favorable hydrophilicity is exhibited.

On the other hand, the content of the phosphate compound in the vapor deposition material is 50 to 98 mass%.

### Examples

The present invention will be described below in further detail with reference to examples and comparative examples. However, the present invention is not limited to these examples.

### (Example 1)

### <Production of vapor deposition material used for forming hydrophilic vapor deposition film>

Hydroxyapatite (white powder particles, average particle size of 10 µm, purity of 98% or more) and cerium oxide (light yellow powder particles, average particle size of 1.35 to 1.75 µm, purity of 99.9%) were weighed out so that a mass proportion of cerium oxide was 10 mass%, and mixing was performed using a 10 L ball mill at a rotational speed of 70 rpm for 30 minutes. After mixing, 15 parts by mass of an organic (acrylic) binder (Ceramo) was added with respect to 100 parts by mass of the mixture, and mixing was performed using a ball mill again for 10 minutes.

Then, a pressure of 400 kgf/cm was applied using a uniaxial pressure molding machine, a molded product was produced and then pulverized, the particle size was adjusted to 1 to 2 mm using a mesh sieve having openings of 1.18 to 2.00, and a granular molded product was obtained. This was put into a 1.125 L alumina crucible, and then heated to 1100°C at a heating speed of 100°C/hour in a baking device, and left for 2 hours, and then cooled to room temperature, and thereby a granular phosphoric acid vapor deposition material was obtained.

### <Formation of hydrophilic vapor deposition film>

A polycarbonate substrate (with a diameter of 74.5 mm, a thickness of 2.0 mm) was placed on a dome using a vacuum evaporation device (with a dome diameter of φ900 mm and a deposition distance of 890 mm), and the phosphoric acid vapor deposition material was placed in the evaporation device. The pressure in the chamber was reduced to 1.0×10⁻³ Pa or less, and while the shutter was closed, an electron beam was emitted to the phosphoric acid vapor deposition material for 3 minutes, and the material was dissolved.

Next, an electron beam with a deposition current 170 mA and an acceleration voltage of 6 kV was applied to the dissolved phosphoric acid vapor deposition material, the vaporized hydrophilic thin film component was deposited on a substrate, and a hydrophilic vapor deposition film with a film thickness of 100 nm was formed. In this case, a deposition rate was 2 Å/sec, and oxygen was introduced into the chamber so that the pressure was 1.2×10⁻² Pa.

The pressure in the vacuum device was returned to atmospheric pressure, and the removed substrate was used as a deposition substrate.

### <Evaluation of hydrophilic performance>

Using a contact angle meter CA-X150 (commercially available from Kyowa Interface Science, Inc.), 2.5 µl of pure water was added dropwise to a film surface of the deposition substrate, and when an image processing type contact angle was measured, it was 4.5°, and it was found that a favorable hydrophilic performance was obtained.

Generally, when the contact angle of pure water is smaller than 90°, this is called hydrophilicity, and when the contact angle is larger than 90°, this is called hydrophobicity. However, in the present invention, in consideration of practicality of the hydrophilic vapor deposition film, when the contact angle of pure water was 60° or less (preferably 50° or less), the film was called a hydrophilic film.

### <Immersion test for deposition substrate>

When the deposition substrate was subjected to component analysis on polycarbonate equipment using a ZSX Primus 2 (X-ray fluorescence analysis (XRF) commercially available from Rigaku Corporation), Ca and Ce were detected in addition to phosphorus and oxygen. After the component analysis, the substrate was immersed in a container filled with pure water for 2 hours, and the substrate was then subjected to component analysis on polycarbonate equipment again using the above XRF, and components of the film remaining on the surface of the substrate were analyzed. After the immersion test, phosphorus, Ca, and Ce were detected, phosphorus remained in the thin film, and components of the hydrophilic vapor deposition film had not dissolved.

### <Outdoor exposure test>

The produced deposition substrate was left outdoors and deionized water was sprayed onto the substrate every day by blowing. The spread of water on the substrate was checked and the durability was evaluated. In this case, evaluation was stopped when the spread of water was not more than 50% in visual observation, and the number of days at that time was recorded. The hydrophilic performance was maintained even after 5 weeks or longer.

### (Examples 2 to 11 and 14 to 17, and Reference Examples 12 and 13 (outside the scope of the claims but useful to understand the invention))

Vapor deposition materials were produced in the same manner as in Example 1 except that the mass proportion of cerium oxide was 2 mass%, and a film was formed on a substrate to obtain a deposition substrate of Example 2. When the hydrophilic performance was evaluated, and the immersion test and the outdoor exposure test were performed, hydrophilic vapor deposition films having high water resistance as in Example 1 were obtained.

In addition, deposition substrates of Examples 2 to 11 and 14 to 17 and Reference Examples 12 and 13 were obtained by changing components of the phosphate compound of raw materials and metal oxide to be added in the production example of Example 1 as shown in Table 2. The evaluation results are shown in Table 2.

### (Example 18)

Using the vapor deposition material obtained in Example 1, a hydrophilic vapor deposition film with a film thickness of 100 nm was formed on a BK-7 glass substrate in the same manner as in Example 1. When the refractive index n of the hydrophilic vapor deposition film was determined using a spectroscopic ellipsometer (EC-400 commercially available from J. A. Woollam), it was n = 1.64 at 550 nm. This thin film was transparent and can be used as an optical thin film in combination with other multilayer films.

Examples of an optical design in which the hydrophilic vapor deposition film was used on the outermost surface of a 17-layer optical mirror are shown in Fig. 1 and Table 3. In addition, optical characteristics of the mirror are shown in Fig. 2. Since the mirror was able to maintain the hydrophilic performance for a long time and did not require ultraviolet light, it could be used for indoor and medical applications.

### (Comparative Example 1)

A vapor deposition material was produced in the same manner as in Example 1 such that aluminum fluoride powder (white powder particles, average particle size of 38.5 µm, purity of 99.9%) was added to calcium phosphate powder used in Example 1 so that the mass proportion thereof was 10 mass%. A deposition substrate was obtained in the same manner as in Example 1 using the obtained vapor deposition material, and the hydrophilic performance was evaluated, and the immersion test and the outdoor exposure test were performed. The initial contact angle was 4.6°, which indicated a favorable hydrophilic performance, but no aluminum was contained in the thin film, and in the outdoor exposure test, the hydrophilic performance was not maintained after one week, and the water resistance was low.

### (Comparative Examples 2 to 7)

Deposition substrates of Comparative Examples 2 to 7 were obtained by changing components of the phosphate compound of raw materials and metal oxide to be added as shown in Table 2. The evaluation results are shown in Table 2.

**[Table 2]**

| | Phosphate compound | Metal compound | Proportion of metal compound added [mass%] | Proportion of trivalent or hexavalent metal element added to material [mass%] | Initial contact angle [°] | Remaining of phosphorus after immersion test | Durability period in outdoor exposure test | Metal element contained in film | (Trivalent or hexavalent metal in film)/ (phosphorous-contai ning metal in film) [mass%] | Amount of phosphorous contained in film [mass%] |
|---|---|---|---|---|---|---|---|---|---|---|
| Example 1 | Calcium phosphate | CeO₂ | 10 | 8.1 | 4.5 | Remained | 5W or longer | Ca, Ce | 8.1 | 82.2 |
| Example 2 | Calcium phosphate | CeO₂ | 2 | 1.6 | 3.9 | Remained | 2W | Ca, Ce | 1.1 | 87.3 |
| Example 3 | Calcium phosphate | CeO₂ | 40 | 32.6 | 33.3 | Remained | 5W or longer | Ca, Ce | 55.2 | 42.2 |
| Example 4 | Calcium phosphate | CeO₂ | 50 | 40.7 | 55.8 | Remained | 5W or longer | Ca, Ce | 79.1 | 19.8 |
| Example 5 | Calcium phosphate | CeF₃ | 10 | 2.9 | 4.6 | Remained | 5W or longer | Ca, Ce | 12.2 | 77.5 |
| Example 6 | Calcium phosphate | CeF₃ | 40 | 11.6 | 10.1 | Remained | 5W or longer | Ca, Ce | 35.7 | 54.9 |
| Example 7 | Calcium phosphate | Fe₂O₃ | 10 | 6.2 | 12.8 | Remained | 5W or longer | Ca, Fe | 17.6 | 67.1 |
| Example 8 | Calcium phosphate | Bi₂O₃ | 10 | 8.4 | 5.1 | Remained | 5W or longer | Ca, Bi | 37.8 | 49.8 |
| Example 9 | Aluminum phosphate | Fe₂O₃ | 10 | 24.5 | 3.4 | Remained | 5W or longer | Fe | 1.4 | 98.6 |
| Example 10 | Aluminum phosphate | Bi₂O₃ | 10 | 23.2 | 21.3 | Remained | 5W or longer | Bi | 9.2 | 90.8 |
| Example 11 | Aluminum phosphate | Ga₂O₃ | 40 | 7.7 | 11.1 | Remained | 5W or longer | Ga | 2.1 | 97.9 |
| Example 12 | Aluminum phosphate | WO₃ | 10 | 7.9 | 6.3 | Remained | 3W | W | 1.4 | 98.6 |
| Example 13 | Aluminum phosphate | WO₃ | 40 | 31.7 | 25.1 | Remained | 2W | W | 6.7 | 93.3 |
| Example 14 | Aluminum phosphate | CeO₂ | 10 | 8.1 | 7.03 | Remained | 5W or longer | Ce | 0.8 | 31.3 |
| Example 15 | Aluminum phosphate | CeO₂ | 40 | 32.6 | 21 | Remained | 5W or longer | Ce | 1.6 | 29.2 |
| Example 16 | Aluminum phosphate | CeF₃ | 10 | 2.9 | 11 | Remained | 5W or longer | Ce | 3.5 | 96.5 |
| Example 17 | Aluminum phosphate | CeF₃ | 40 | 11.6 | 18.4 | Remained | 5W or longer | Ce | 6.5 | 93.5 |
| Comparative Example 1 | Calcium phosphate | AlF₃ | 10 | 6.8 | 4.6 | Not more than detection limit | 1W | Ca | - | - |
| Comparative Example 2 | Calcium phosphate | - | 0 | 0.0 | 4.8 | Not more than detection limit | 1W | Ca | - | - |
| Comparative Example 3 | Calcium phosphate | TiO₂ | 9 | 5.4 | 17.4 | Not more than detection limit | 1W or shorter | Ca | - | - |
| Comparative Example 4 | Calcium phosphate | SiO₂ | 9 | 4.2 | 5.3 | Not more than detection limit | 1W or shorter | Ca | - | - |
| Comparative Example 5 | Aluminum phosphate | - | 0 | 0.0 | 5.7 | Not more than detection limit | 1W | Not more than detection limit | | |
| Comparative Example 6 | Aluminum phosphate | AlF₃ | 10 | 3.2 | 4.9 | Not more than detection limit | 1W | Notmore than detection limit | - | - |
| Comparative Example 7 | Aluminum phosphate | ZnO | 10 | 8.0 | 6.5 | Not more than detection limit | 1W | Zn | - | - |

**[Table 3]**

| Layer | Material | index | Optical Thickness[FWOT] | Physical thick.(nm) |
|---|---|---|---|---|
| - | air | | | - |
| 17 | Example 1 | 1.64 | 0.250 | 91.5 |
| 16 | SiO₂ | 1.46 | 0.250 | 102.9 |
| 15 | TiO₂ | 2.29 | 0.250 | 65.5 |
| 14 | SiO₂ | 1.46 | 0.250 | 102.9 |
| 13 | TiO₂ | 2.29 | 0.250 | 65.5 |
| 12 | SiO₂ | 1.46 | 0.250 | 102.9 |
| 11 | TiO₂ | 2.29 | 0.250 | 65.5 |
| 10 | SiO₂ | 1.46 | 0.250 | 102.9 |
| 9 | TiO₂ | 2.29 | 0.250 | 65.5 |
| 8 | SiO₂ | 1.46 | 0.250 | 102.9 |
| 7 | TiO₂ | 2.29 | 0.250 | 65.5 |
| 6 | SiO₂ | 1.46 | 0.250 | 102.9 |
| 5 | TiO₂ | 2.29 | 0.250 | 65.5 |
| 4 | SiO₂ | 1.46 | 0.250 | 102.9 |
| 3 | TiO₂ | 2.29 | 0.250 | 65.5 |
| 2 | SiO₂ | 1.46 | 0.250 | 102.9 |
| 1 | TiO₂ | 2.29 | 0.250 | 65.5 |
| Substrate | BK7 | 1.52 | - | - |

### [Reference Signs List]

1: Optical multilayer film, 2: Substrate

## Claims

1. A hydrophilic vapor deposition film comprising at least phosphorus, oxygen, and two or more metal elements as constituent elements,
wherein at least one of the metal elements is a metal element capable of becoming trivalent,
wherein a content of the metal element capable of becoming trivalent is 0.7 to 80.0 mass% based on a total amount of phosphorus and metal elements contained in the hydrophilic vapor deposition film, and
wherein the content of phosphorous in the hydrophilic vapor deposition film is 19.0 to 99.0 mass%,
**characterized in that**
the metal element capable of becoming trivalent is at least one selected from the group consisting of cerium, iron, gallium, and bismuth.

2. A vapor deposition material for forming a vapor deposition film in a vapor deposition process comprising at least phosphorus, oxygen,
and two or more metal elements as constituent elements,
wherein at least one of the metal elements is a metal element capable of becoming trivalent,
wherein the vapor deposition material comprises a phosphate compound, and
a content of the phosphate compound in the vapor deposition material is 50 to 98 mass %,
**characterized in that**
a compound containing the metal capable of becoming trivalent is at least one selected from the group consisting of a cerium compound, an iron compound, a gallium compound, and a bismuth compound.

3. The vapor deposition material according to claim 2,
wherein a content of the metal element capable of becoming trivalent is 1.5 to 42.0 mass% based on a total amount of phosphoric acid and metal elements contained in the vapor deposition material.

4. A method of producing a hydrophilic vapor deposition film, the method comprising
a process of vaporizing a vapor deposition material and depositing the material on a substrate,
wherein the vapor deposition material is the vapor deposition material according to any one of claims 2 or 3.

5. A multilayer film comprising a hydrophilic vapor deposition film on a substrate,
wherein the hydrophilic vapor deposition film is formed on the outermost surface, and
the hydrophilic vapor deposition film is the hydrophilic vapor deposition film according to any one of claim 1.

## Patentansprüche

1. Hydrophiler Dampfabscheidungsfilm, umfassend mindestens Phosphor, Sauerstoff und zwei oder mehr Metallelemente als konstituierende Elemente, wobei mindestens eines der Metallelemente ein Metallelement ist, das dreiwertig werden kann,
wobei ein Gehalt des Metallelements, das dreiwertig werden kann, 0,7 bis 80,0 Massen-% beträgt, bezogen auf die Gesamtmenge an Phosphor und Metallelementen, die in dem hydrophilen Dampfabscheidungsfilm enthalten sind, und
wobei der Gehalt an Phosphor im hydrophilen Dampfabscheidungsfilm 19,0 bis 99,0 Massen-% beträgt,
**dadurch gekennzeichnet, dass**
das Metallelement, das dreiwertig werden kann, mindestens eines ist, das aus der Gruppe ausgewählt ist, die aus Cer, Eisen, Gallium und Bismut besteht.

2. Dampfabscheidungsmaterial zur Bildung eines hydrophilen Dampfabscheidungsfilms in einem Dampfabscheidungsverfahren, das mindestens Phosphor, Sauerstoff und zwei oder mehr Metallelemente als konstituierende Elemente enthält,
wobei mindestens eines der Metallelemente ein Metallelement ist, das dreiwertig werden kann,
wobei das Dampfabscheidungsmaterial eine Phosphatverbindung umfasst, und
ein Gehalt der Phosphatverbindung im Dampfabscheidungsmaterial 50 bis 98 Massen-% beträgt,
**dadurch gekennzeichnet, dass**
eine Verbindung, die das Metall enthält, das dreiwertig werden kann, mindestens eine Verbindung ist, die aus der Gruppe ausgewählt ist, die aus einer Cerverbindung, einer Eisenverbindung, einer Galliumverbindung und einer Bismutverbindung besteht.

3. Dampfabscheidungsmaterial nach Anspruch 2,
wobei ein Gehalt des Metallelements, das dreiwertig werden kann, 1,5 bis 42,0 Massen-% beträgt, bezogen auf die Gesamtmenge an Phosphorsäure und Metallelementen, die in dem Dampfabscheidungsmaterial enthalten sind.

4. Verfahren zur Herstellung eines hydrophilen Dampfabscheidungsfilms, wobei das Verfahren umfasst
ein Verfahren zum Verdampfens eines Dampfabscheidungsmaterials und Abscheiden des Materials auf einem Substrat,
wobei das Dampfabscheidungsmaterial das Dampfabscheidungsmaterial nach einem der Ansprüche 2 oder 3 ist.

5. Mehrschichtiger Film, umfassend einen hydrophilen Dampfabscheidungsfilm auf einem Substrat,
wobei der hydrophile Dampfabscheidungsfilm auf der äußersten Oberfläche gebildet ist, und
der hydrophile Dampfabscheidungsfilm der hydrophile Dampfabscheidungsfilm nach einem der Ansprüche 1 ist.

## Revendications

1. Film de dépôt en phase vapeur hydrophile comprenant au moins du phosphore, de l'oxygène, et deux ou plus de deux éléments métalliques en tant qu'éléments constitutifs,
dans lequel au moins l'un des éléments métalliques est un élément métallique capable de devenir trivalent,
dans lequel la teneur en l'élément métallique capable de devenir trivalent est de 0,7 à 80,0 % en masse par rapport à la quantité totale de phosphore et d'éléments métalliques contenus dans le film de dépôt en phase vapeur hydrophile, et
dans lequel la teneur en phosphore du film de dépôt en phase vapeur hydrophile est de 19,0 à 99,0 % en masse,
**caractérisé en ce que** l'élément métallique capable de devenir trivalent est au moins l'un choisi dans le groupe constitué par le cérium, le fer, le gallium, et le bismuth.

2. Matériau de dépôt en phase vapeur pour former un film de dépôt en phase vapeur dans un procédé de dépôt en phase vapeur comprenant au moins du phosphore, de l'oxygène, et deux ou plus de deux éléments métalliques en tant qu'éléments constitutifs,
dans lequel au moins l'un des éléments métalliques est un élément métallique capable de devenir trivalent,
dans lequel le matériau de dépôt en phase vapeur comprend un composé phosphate, et
la teneur en le composé phosphate du matériau de dépôt en phase vapeur est de 50 à 98 % en masse,
**caractérisé en ce qu'**un composé contenant le métal capable de devenir trivalent est au moins l'un choisi dans le groupe constitué par un composé du cérium, un composé du fer, un composé du gallium, et un composé du bismuth.

3. Matériau de dépôt en phase vapeur selon la revendication 2, dans lequel la teneur en l'élément métallique capable de devenir trivalent est de 1,5 à 42,0 % en masse par rapport à la quantité totale d'acide phosphorique et d'éléments métalliques contenus dans le matériau de dépôt en phase vapeur.

4. Procédé de production d'un film de dépôt en phase vapeur hydrophile, le procédé comprenant
un traitement de vaporisation d'un matériau de dépôt en phase vapeur et de déposition du matériau sur un substrat,
dans lequel le matériau de dépôt en phase vapeur est le matériau de dépôt en phase vapeur selon l'une quelconque des revendications 2 et 3.

5. Film multicouche comprenant un film de dépôt en phase vapeur hydrophile sur un substrat,
dans lequel le film de dépôt en phase vapeur hydrophile est formé sur la surface la plus extérieure, et
le film de dépôt en phase vapeur hydrophile est le film de dépôt en phase vapeur hydrophile selon la revendication 1.
